(19) Europäisches Patentamt
European Patent Office
Office européen des brevets

(11) **EP 3 552 591 B1**

(12) **EUROPEAN PATENT SPECIFICATION**

(45) Date of publication and mention
of the grant of the patent:
**27.09.2023 Bulletin 2023/39**

(21) Application number: **18167187.6**

(22) Date of filing: **13.04.2018**

(51) International Patent Classification (IPC):
***A61F 13/534*** *(2006.01)*    ***A61F 13/15*** *(2006.01)*
***A61F 13/53*** *(2006.01)*

(52) Cooperative Patent Classification (CPC):
**A61F 13/53409; A61F 13/15707; A61F 13/53418;
A61F 13/53427;** A61F 2013/530437

(54) **ABSORBENT CORE, ARTICLES COMPRISING SAID CORE, AND METHODS OF MAKING**

SAUGFÄHIGER KERN, ARTIKEL MIT BESAGTEM KERN UND VERFAHREN ZUR HERSTELLUNG

NOYAU ABSORBANT, ARTICLES COMPRENANT LEDIT NOYAU ET PROCÉDÉS DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**16.10.2019 Bulletin 2019/42**

(73) Proprietors:
• **Ontex BV
9255 Buggenhout (BE)**
• **Ontex Group NV
9320 Erembodegem (BE)**

(72) Inventor: **WEBER, Ainas
53474 Bad Neuenahr-Ahrweiler (DE)**

(74) Representative: **Saurat, Thibault
Ontex BV
Legal Department
Korte Keppestraat 21
9320 Erembodegem (BE)**

(56) References cited:
**GB-A- 2 174 037**     **US-A- 2 852 026
US-A1- 2005 113 776**

**Description**

**TECHNICAL FIELD**

The disclosure pertains to the technical field of absorbent personal hygiene products. In particular, the present disclosure relates to an absorbent core that can be used within an article for absorbing body fluids and exudates, such as urine and fecal material, or blood, menses, and vaginal fluids. More particularly, the present disclosure relates to absorbent articles, such as disposable diapers or diaper pants, disposable incontinence diapers or pants, and which are configured to collect and contain fecal material and avoid leakage, or sanitary napkins or panty liners, which are configured to collect and contain blood, menses, urine, vaginal fluids and avoid leakage.

**BACKGROUND**

Absorbent articles for personal hygiene such as disposable diapers, feminine protection pads and adult incontinence undergarments, are designed to absorb and contain body exudates, in particular but not limited to urine. These absorbent articles usually comprise several layers having different functions, for example a topsheet, a backsheet and inbetween an absorbent core, among other layers. The absorbent core's function is to absorb and retain the exudates for a prolonged amount of time, for example overnight for a diaper, minimize rewet to keep the wearer dry and avoid soiling of clothes or bed sheets.

Absorbent cores can expand several times their initial volumes when wet. It is desirable that the cores in this expanded state maintain their structural integrity and do not break or burst even when submitted to a shock such as a child sitting heavily on his diaper. It is generally also desirable that absorbent cores should be thin (at least when dry) and require as little material as possible for costs and environmental reasons.

The majority of currently marketed absorbent cores for diapers however comprise as absorbent material a blend of comminuted wood pulp (fluff) with superabsorbent polymer particles (SAP), also called absorbent gelling materials (AGM), see for example US 5,151,092. Absorbent articles having a core consisting of essentially SAP as absorbent material (so called "airfelt-free" or "fluff-free" or "fluffless" cores) have also been proposed but are less common than traditional mixed cores (see e.g. WO2008/155699).

US2008/0312621 and US2008/0312622 describe a disposable absorbent article comprising a chassis including a topsheet and a backsheet, a substantially cellulose free absorbent core located between the topsheet and the backsheet and having a wearer facing side oriented toward a wearer when the article is being worn and an opposed garment facing side, and a liquid acquisition system comprising chemically cross-linked cellulosic fibers disposed between the liquid permeable topsheet and the wearer facing side of the absorbent core. The liquid acquisition system may comprise a nonwoven as an upper acquisition layer and a lower acquisition layer which may comprise chemically cross-linked cellulose fibers. In such a design the lower acquisition layer contacts the upper substrate of the cellulose free absorbent core. A typical process to make such a liquid acquisition system is airlaying the unbound chemically cross-linked cellulose fibers onto the upper acquisition layer nonwoven: in this case, the upper acquisition layer nonwoven always fully covers the lower acquisition layer comprising the chemically cross-linked cellulose fibers.

It is common in the art to provide anatomical and/or shaped cores that have a narrowing at the crotch region, as for example disclosed in US2013/331806A as this provides better comfort to the user in the leg hole regions. However, such cores are typically made by forming such anatomical shape directly during the drum laying step of the core formation process.

It is also known to provide higher absorbency of the core in specific regions of the crotch region, as exemplified in US2014/163501, to thus provide better fluid handling in regions where it is most needed. However this is typically done by using complex processing for accurate metering of SAP and/or channel formation during the drum forming process.

Documents GB2174037 and US2852026 describe methods for making anatomical absorbent cores.

There thus still remains a need to provide a simpler and more cost effective process of forming cores that are both anatomically shaped and at the same time provide the desired graded absorbency, at a much reduced cost than what currently available on the market.

**SUMMARY OF THE INVENTION**

In a first aspect, the disclosure relates to a process for making an anatomical absorbent core as defined in Claim 1.

In a second aspect, the disclosure relates to a process for making an absorbent article comprising the steps of: making an anatomical absorbent core; providing a first web of material; providing a second web and/or film of material, wherein said first web is liquid permeable and said second web and/or film is liquid impermeable; conveying said core, said first web and said second web and/or film along a machine direction to a joining station; joining said core to said first web and said second web and/or film to form a laminate.

In a third aspect, the disclosure relates to an anatomical absorbent core as defined in claim 7. Said absorbent core comprises: a front portion; a back portion ; and an intermediate portion extending between the front and back portions, wherein said intermediate portion is defined by at least, preferably only, two voids also positioned between the front and back portions and extending outboard of the intermediate portion on opposite sides thereof, wherein the entire anatomical absorbent core is capable of absorbing and retaining liquid therein and wherein front and back portions have a first and second absorption capacity per unit area respectively and the intermediate portion has a third absorption capacity per unit area, as measured according to ISO 11948-1, and wherein the first and second absorption capacities are substantially the same and the third absorption capacity per unit area is at least about two times greater than the first absorption capacity per unit area.

In a further aspect, the disclosure relates to absorbent articles comprising anatomical cores as described herein.

In a further aspect of the disclosure the sheet-like layer as described herein comprises superasbsorbent polymer in the form of superabsorbent polymer particles (SAP) or superabsorbent polymer fibers (SAF), preferably consists of SAF. When SAF is used, said fibers are formed into an nonwoven substrate forming the sheet-like layer itself. By the term "superabsorbent polymer fibers" as used herein, is meant fibers made from superabsorbent polymers (as opposed to particles made therefrom). Examples of suitable such SAF fibers for use herein are selected from those of Example 1, Example 2, Example 3, and/or Example 4 (page 5, lines 1-46) of EP3190216A1. Said fibers typically being used to form nonwoven webs or substrates according to the same referenced application.

In all embodiments of the present disclosure, the cutting and folding described herein is typically applied only on the absorbent sheet-like/form layer (i.e. the core of the absorbent article) and no other portions of the absorbent article are so cut and folded.

## DESCRIPTION OF FIGURES

Fig. 1 illustrates a schematic of a process according to an embodiment of the disclosure.

Fig. 2 illustrates a top view schematic of an absorbent core according to an embodiment of the disclosure.

Fig. 3A illustrates a cross-section schematic taken about axis x-x of Fig. 2 according to an embodiment of the disclosure.

Fig. 3B illustrates a cross-section schematic taken about axis x-x of Fig. 2 according to an embodiment of the disclosure.

Fig. 3C illustrates a cross-section schematic taken about axis x-x of Fig. 2 according to an embodiment of the disclosure.

Fig. 4 illustrates a cross-section schematic showing a folding arrangement according to an embodiment of the disclosure.

Fig. 5 is a schematic top view (in its laid-flat state) of an absorbent article according to an embodiment of the present disclosure.

Fig. 6A-F show pictures of exemplary embodiments according to embodiments of the disclosure (cutting pattern and shape) and folded core.

Fig. 7A-C show pictures of exemplary embodiments according to embodiments of the disclosure (cutting pattern and shape) and folded core.

Fig. 8A-B show pictures of an exemplary embodiment according to an aspect of the disclosure having an exudate-receiving basin.

Fig. 9 shows a picture of a folded core according to an aspect of the disclosure further comprising lateral flaps for improved fit.

Fig. 10 shows a schematic representation of a cutting and folding station according to an aspect of the present disclosure.

## DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation or element referred to.

The recitation of numerical ranges by endpoints includes all numbers and fractions subsumed within that range, as well as the recited endpoints, except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

Preferably, a diaper comprises a liquid permeable "top sheet", a liquid impermeable "back sheet", and an "absorbent medium or core" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressuresensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, the term "associated or joined or adhered" encompasses, unless expressly stated, configurations in which e.g. a top sheet is directly joined to a back sheet by affixing the top sheet directly to the back sheet, and also configurations wherein the top sheet is joined to the back sheet by affixing the top sheet to intermediate members which in turn are affixed to the back sheet (i.e. indirectly joining). Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The terms "back portion", "back section" and "rear back section" are used herein as synonyms and refer to the area of the absorbent article which is contact with the back of the wearer when the absorbent article is worn.

The term "backsheet" refers to a material forming a liquid impermeable cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "front portion", "belly section" and "front belly section" are used herein as synonyms and refer to the area of the absorbent article which is contact with the belly of the wearer when the absorbent article is worn.

As used herein, the "body-facing" or "bodyside" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/$m^2$ - 24 hours.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

An absorbent article can comprise leg containment gaskets (also broadly referred to as "cuffs"). Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

"Hotmelt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. For example, a typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

As used herein the term "extensible" means elongatable in at least one direction, but not necessarily recoverable.

The term "finished" or "final", when used with reference to a product, means that the product has been suitably manufactured for its intended purpose.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" or "fully stretched state" or "extended state" is intended to refer to the article when it is flattened into a plane or is substantially flattened into a plane and is used in contrast to when the article otherwise positioned, such as when the article is folded or shaped in or for use by a wearer.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to 100% by weight. Typically, the superabsorbent material, when present, will be included in an amount of from about 30% to about 70% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 $\mu$m, preferably between 300 to 600 $\mu$m, more preferably between 400 to 500 $\mu$m.

The term "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction (i.e. is parallel to the x-axis).

"Substantially perpendicular (or parallel)" refers to an element extending at an angle of not more than 35°, preferably less than 25°, more preferably less than 20°, even more preferably less than 15°, even more preferably less than 10°, most preferably less than 5°, from the referred perpendicular (or parallel) axis.

By the term "distinct cellulosic fibers" as used herein, is meant cellulosic fibers that are not part of a substrate (e.g. a nonwoven layer) and are rather distinct thereof and/or physically separated therefrom (i.e. what is typically referred to as fluff, fluff-pulp, or tow fibers), and typically are in the form of cellulosic fibers that are for example enclosed though kept separate from said substrate (i.e. not integral). For sake of clarity, cellulosic fibers that may be present within and/or integral to a substrate (e.g. a cellulosic nonwoven layer) are not encompassed within its meaning.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 5-60 $\mu$m, preferably from 10-30 $\mu$m. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

"Absorption capacity per unit area", also referred to as "absorbency", as used herein means absorption capacity as measured according to ISO 11948-1 [known also as "the Rothwell method"] divided by the surface area (taken about a plane parallel to both the length, being parallel to the machine direction MD, and the width of the sheet-like layer) of the component referred to. For example, the absorption capacity per unit area of the intermediate portion is the absorption capacity of the intermediate portion according to the test method procedure of ISO 11948-1 divided by the surface area of said intermediate portion, with unit of g/cm$^2$.

Embodiments according to the disclosure will now be described. It is understood that technical features described in one or more embodiments maybe combined with one or more other embodiments without departing from the intention of the disclosure and without generalization therefrom.

THE ABSORBENT CORE

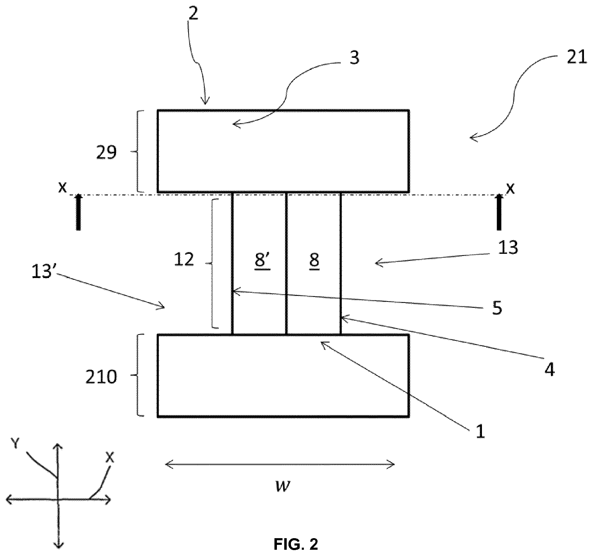

In an embodiment, as shown in exemplary Fig.2 to 4, the absorbent core **21** obtainable from (preferably obtained directly and/or made according to) the below described processes comprises: a front portion **29**; a back portion **210**; and an intermediate portion **12** extending between the front and back portions **29, 210**, wherein said intermediate portion **12** is defined by at least, preferably only, two voids **13, 13'** also positioned between the front and back portions **29, 210** and extending outboard of the intermediate portion **12** on opposite sides thereof, wherein the entire anatomical absorbent core **21** is capable of absorbing and retaining liquid therein and wherein front and back portions **29, 210** have a first and second absorption capacity per unit area respectively and the intermediate portion **12** has a third absorption capacity per unit area, as defined herein above, and wherein the first and second absorption capacities are substantially the same and the third absorption capacity per unit area is at least about 2, preferably at least about 2.5, more preferably at least about 3, times greater than the first absorption capacity per unit area. Advantageously, the entire core has a base absorbency and only where needed most, the core has a substantially higher absorbency.

Preferably, the thickness of the intermediate portion **12** is at least about twice, preferably at least three times, the thickness of the front and back portions **29, 210**, as measured according to ISO 9073-2 [method A in particular]. Increasing absorbency by simply folding (and increasing the thickness) of the central crotch region has been found to be very costeffective.

Typically the intermediate portion **12** does not extend beyond the voids **13,13'** along the length of the absorbent core (parallel to the machine direction **MD**). It has been found that having an abrupt demarcation between the intermediate portion **12** and each of the front and back portions **29, 210** folding lines are formed in the transverse direction that help shaping of said front and back portions and improve fluid handling.

Preferably, the core is composed of a sheet-form layer **1** of absorbent material comprising fibers (typically selected from the group consisting of polyester fibers, polyethylene terephthalate (PET) fibers, polyethylene (PE) fibers, polypropylene (PP) fibers, cellulose fibers, and mixtures thereof, most preferably cellulose fibers) and/or superabsorbent polymer, said sheet-form layer **1** comprising oppositely disposed top and bottom surfaces **2, 3**, first and second longitudinal sides **4, 5** and a width **w** extending between said sides **4, 5**, and wherein portions of the first and second longitudinal sides **4, 5** are folded over the top or bottom surface **2, 3** of the sheet-form layer **1** to form the intermediate portion **12**, preferably wherein said portions of the first and second longitudinal sides **4, 5** are folded over said top or bottom surface **2, 3** a plurality of times, typically wherein said portions are the intermediate flaps **8, 8'**. An advantage of this arrangement is to further increase absorption capacity per unit area without adding any further material and further limiting scrap.

Preferably, the sheet-form layer **1** comprises, preferably consists of, compressed cellulose fibers and immobilized superabsorbent polymer particles therein and/or thereon. Such material can be easily and cheaply made into a continuous sheet of material that can be rolled up into reels for subsequent processing (sometimes also referred to as "SAP paper") according to the processes described herein below.

In a preferred embodiment, the anatomical absorbent core **21** has a perimeter shape selected from substantially H-shaped, T-shaped or hour-glass shaped.

Preferably, the sheet-form layer **1** comprises, preferably consists of, a nonwoven carrier (that may selected from the group consisting of polyester fibers, polyethylene terephthalate (PET) fibers, polyethylene (PE) fibers, polypropylene (PP) fibers, cellulose fibers, and mixtures thereof, most preferably cellulose fibers) and superabsorbent polymer particles immobilized therein and/or thereon, preferably wherein the carrier comprises a nonwoven top layer, penetrable by the particles and a nonwoven bottom layer, said top layer being mechanically bonded by hydroentanglement to the bottom layer, wherein said carrier, the bottom layer and the top layer are hydrophilic, and wherein the bottom layer is porous with a pore size smaller than said particles. Such has been found to allow extremely good absorption performance while limiting the thickness of the sheet and thus also of the resulting folded core.

Preferably, the top layer is a carded nonwoven made of staple fibers which are hydroentangled to the bottom layer and wherein the bottom layer is a spunbond meltblown spunbond fabric. This arrangement has been found particularly useful to ensure that the SAP is securely immobilized and is not displaced during the high speed production process.

In a most preferred embodiment, the anatomical absorbent core **21** described herein is free of fluff (also referred to as pulp-fluff) or distinct cellulosic fibers.

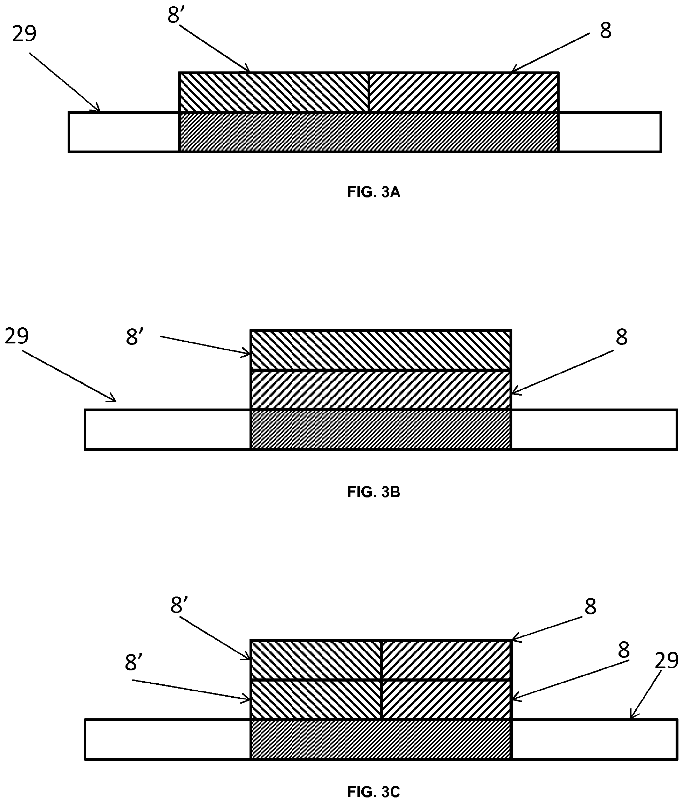

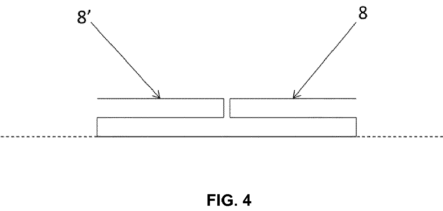

As exemplified in Fig. 3A, the flaps **8, 8'** may be folded such that they abut each other, or can be folded one on top of the other (Fig.3B), or can be folded on themselves (Fig.3C). Preferably, the flaps **8, 8'** are folded on themselves such to form a Z-fold, as exemplified on Fig.4. An advantage of this arrangement is that further absorption capacity per unit area is added to the central portion of the crotch section.

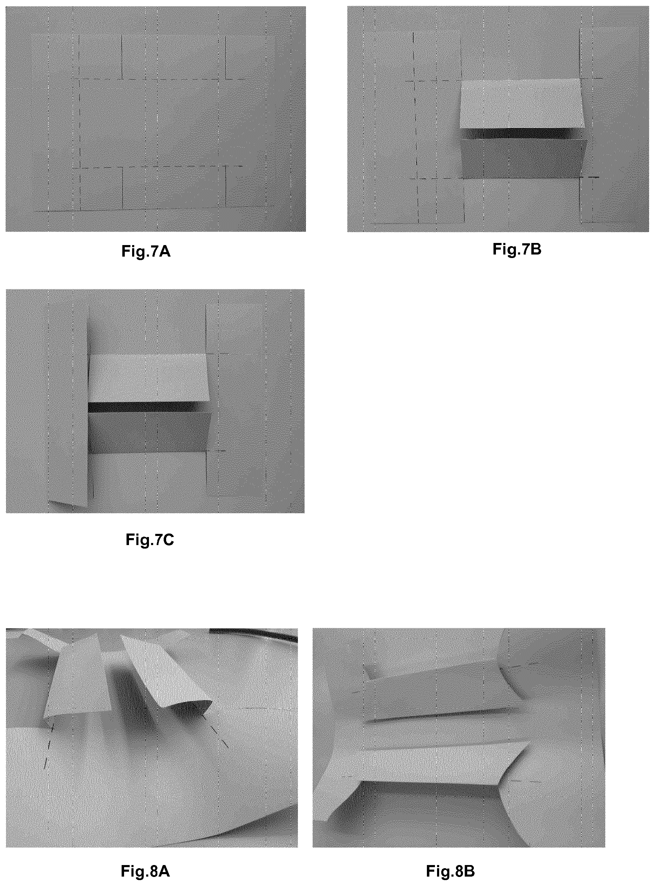

Preferably, the intermediate flaps **8, 8'** are folded such that an exudate-retaining basin is formed between the top or bottom surface **2, 3** of the intermediate portion **12** of the sheet-form layer **1** and the top or bottom surface of the intermediate flaps **8, 8'** respectively. An exemplary core formed by such process arrangement is shown on Fig. 8A-B. Advantageously such allows the formation of a pocket for exudate, especially liquid stool, collection.

In a preferred embodiments, cores described herein above are not enclosed within a core wrap and are preferably directly laminated between a liquid impermeable backsheet and a further liquid permeable layer that may be selected from an acquisition distribution layer (ADL), a topsheet and combinations thereof. Typically core wraps are used to enclose the absorbent layer therein and are made of common materials such as tissue and/or nonwoven webs the purpose of which is to retain the core therein prior to laminating with other components such as topsheet and backsheet to form the absorbent article. It has been found that the core wrap may inhibit full exploitation of the benefits of the core arrangements described herein, such as the formation of an exudate retaining basin, by rather holding the flaps in a folded configuration in both dry and wet conditions and preventing partial unfolding which has rather been found beneficial in the present disclosure.

THE ABSORBENT ARTICLE

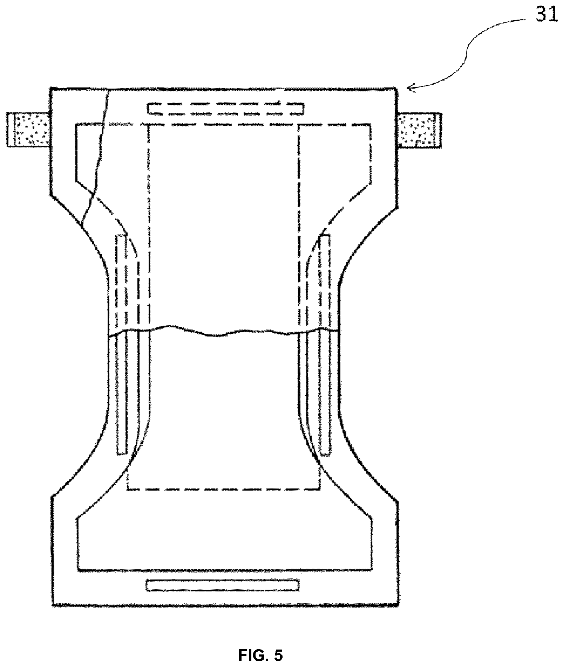

As exemplified in Fig.5, the absorbent article **31** may comprise an anatomical absorbent core **21** as described herein, wherein the super absorbent polymer particles have a size distribution and being dispersed in the carrier according to their particle size distribution gradient along a depth direction or z-direction of said absorbent core, and wherein the smaller super absorbent polymer particles are located on a garment-facing side of the absorbent article and the larger super absorbent polymer particles are located on the opposite side of the absorbent article on a body-facing side thereof. An advantage of this arrangement is that graded absorbency is further achieved in the thickness direction of the core.

The absorbent article **31** may further comprise other known components in the art such as but not limited to longitudinal cuffs, transversal cuffs, elastic waistband (front and/or back), elastic or non-elastic side panels (front and/or back), frontal tape (or landing zone, sometimes referred to as female mechanical fastener or looped fastener) typically joined to the front garment facing surface of the belly section, back tapes (or male fastener with hooks and/or adhesive) typically joined to each of the back side panels, leg elastics, wetness indicator, and the like.

Preferably, the intermediate flaps (8, 8') are folded such that an exudate-retaining basin is formed in the core between the top or bottom surface (2, 3) of the intermediate portion (12) of the sheet-form layer (1) and the top or bottom surface of the intermediate flaps (8, 8') respectively. An exemplary core formed by such process arrangement is shown on Fig. 8A-B. Advantageously such allows the formation of a pocket for exudate, especially liquid stool, collection. In this embodiment, preferably the core is sandwiched between a liquid impermeable backsheet and a liquid permeable topsheet, wherein the topsheet comprises macro-apertures (typically of 100 to 3000 microns, preferably 500 to 2000 microns in diameter) arranged to allow passage of liquid stool therethrough. An advantage of the combination of the basin arrangement and the macro-apertured topsheet is that stool is allowed to be collected within the basin away from the skin of the wearer where it is then absorbed by the core thus improving comfort and reducing the risk of skin rash.

THE PROCESS

The disclosure herein further contemplates a process of making absorbent cores and absorbent articles described herein.

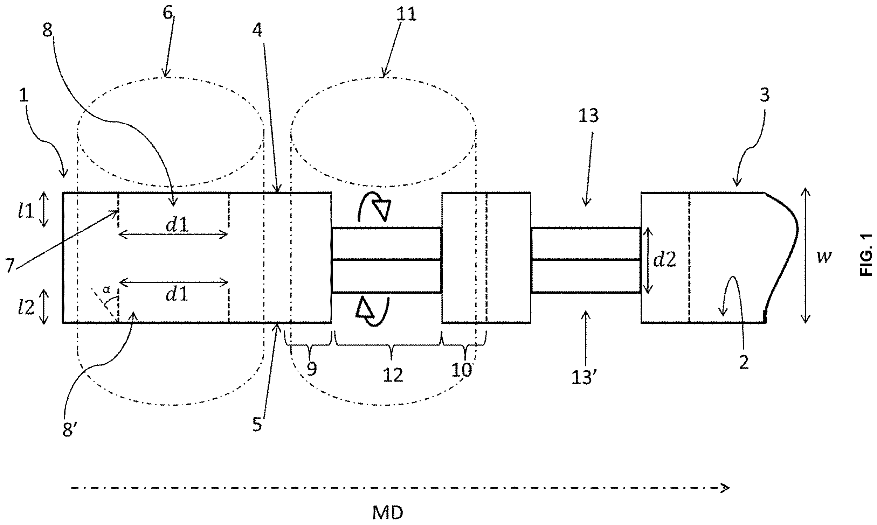

As exemplarily shown in Fig.1, the process for making an anatomical absorbent core according to the present disclosure, comprises the steps of: providing a continuous sheet-form layer **1** of absorbent material comprising fibers (preferably selected from the group consisting of polyester fibers, polyethylene terephthalate (PET) fibers, polyethylene (PE) fibers, polypropylene (PP) fibers, cellulose fibers, and mixtures thereof, most preferably cellulose fibers) and/or superabsorbent polymer, said sheet-form layer **1** comprising oppositely disposed top and bottom surfaces **2, 3**, and first and second longitudinal sides **4, 5** and a width **w** extending between said sides **4, 5**, said sides **4, 5** extending substantially parallel to each other throughout the entire sheet-form layer **1** length extending along a machine direction **MD**; advancing said sheet-form layer **1** along a machine direction **MD** to a cutting station **6**; applying at least two cuts **7** to said sheet-form layer **1** at each of the first and second longitudinal sides **4, 5** along a cutting axis being substantially perpendicular to the machine direction **MD** and parallel to said width **w**, wherein the each of said cuts **7** is spaced apart by a distance **d1** along a longitudinal axis running parallel to the machine direction **MD** and perpendicular to said width **w**, said cuts **7** forming intermediate flaps **8, 8'** between upstream and downstream portions **9, 10** of said sheet-form layer **1**, wherein said cutting axis extends only parallel to said width **w** or at an and angle α therefrom such that no cuts are applied that extend parallel to the machine direction **MD;** advancing said sheet-form layer **1** along a machine direction **MD** to a folding station **11**; folding said intermediate flaps **8, 8'** over the top or bottom surface **2, 3** of the sheet-form layer **1** to form an intermediate portion **12** comprising two or more voids **13, 13'** oppositely disposed on each of the longitudinal sides **4, 5** and spaced apart by a distance **d2** along the width **w**; optionally adhering (preferably by adhesive or mechanical bonding and typically with a continuous or discontinuous bonding pattern) the intermediate flaps **8, 8'** to at least a portion of said top or bottom surface **2, 3**; optionally severing the sheet-form layer **1** along the entire width **w** between each two consecutive voids **13, 13'** positioned along the same first or second longitudinal side **4, 5** to form individual anatomical absorbent cores. It has been advantageously found that such process allows to form anatomical cores with increased absorption in the central crotch region in a cost effective manner. A further advantage of this arrangement is that the absorbent sheet-form layer may be manufactured in a separate process (e.g. by a raw material supplier) and rolled into reels, that can be unwound and processed according to the above described steps. This has the further advantage of no longer needing a drum forming section in the production line and thus also reduces floor space usage and energy consumption as well as increasing production speeds. A further advantage is that the sheet of absorbing material is fully utilized with no scrap being generated in the core formation.

Preferably, each intermediate portion **12** is separated by at least one of the upstream and downstream portions **9, 10** of said sheet-form layer **1** such that the intermediate flaps **8, 8'** are intermittently formed along the machine direction **MD** (i.e. said flaps do not continuously extend along the machine direction **MD** of said layer). Such ensures abrupt demarcations are formed therebetween forming folding lines as described herein above.

In an embodiment, the cutting axis is at an angle α to the machine direction **MD**, typically said angle α being from 25° to 90°, more preferably from 30° to 85°, even more preferably from 35° to 80°, even more preferably from 40° to 75°, most preferably from 45° to 70°. An advantage of cutting at an angle is that a more hourglass shaped core can be achieved rather than solely H-shaped, however applying a cut below the lower limit has the disadvantage of reducing production speeds (bottleneck at the folding station) and/or increasing the risk of tearing and damaging the core during the folding step.

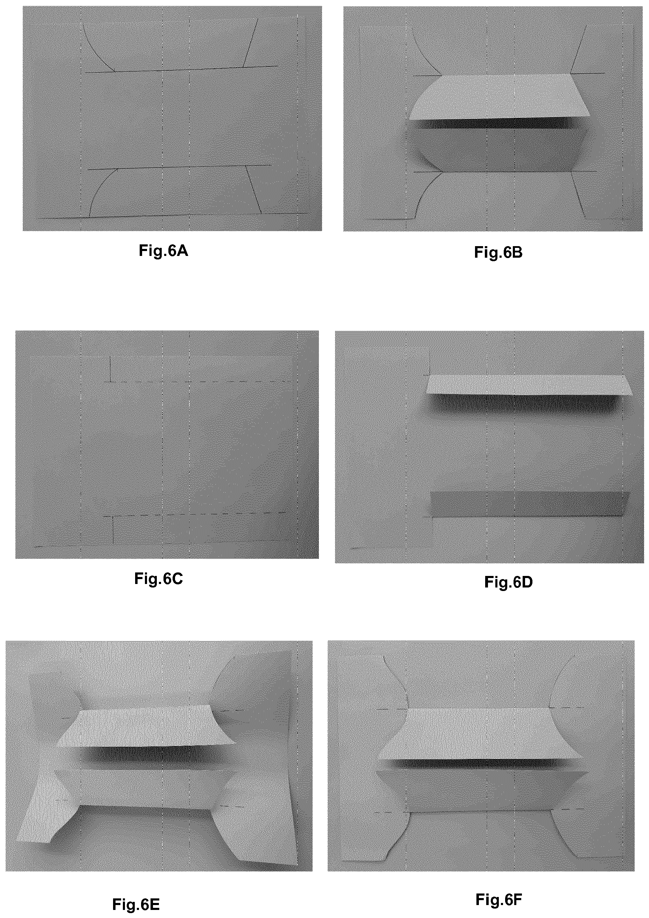

The cuts may be linear (as shown in Fig. 1) or may be curved, or a combination of the two (such as shown in Fig. 6B, E and F). An advantage is that multiple anatomical shapes of the core are enabled.

Preferably, each cut **7** applied on the first longitudinal side **4** has a first cut length **l1** and each cut applied on the second longitudinal side **5** has a second cut length **l2** and wherein the following formula is satisfied:

$$l1 + l2 \geq d2$$

An advantage of the above arrangement is that it permits to achieve a multiplier of original absorbency when later folded vs the flat sheet, and this where it is most needed.

In an embodiment, the folded intermediate flaps **8, 8'** substantially abut each other when in the folded position. Alternatively, the folded intermediate flaps **8, 8'** substantially overlap one another when in the folded position. This arrangement allows for the anatomical shape being formed as well as the graded absorption capacity per unit area.

In an alternative, embodiment, the folded intermediate flaps **8, 8'** are spaced apart along the width direction **w** (i.e. do not abut each other) such that a channel is formed in the intermediate portion **12**, preferably only in the intermediate portion **12**. It has been found that such a channel improves initial fluid burst distribution, however it is desirable for it not to extend into the front and back portions of the absorbent core hence intermittent channels along the machine direction **MD** are rather desirable.

Preferably, the intermediate flaps **8, 8'** are folded such that an exudate-retaining basin is formed between the top or bottom surface **2, 3** of the intermediate portion **12** of the sheet-form layer **1** and the top or bottom surface of the intermediate flaps **8, 8'** respectively. An exemplary core formed by such process arrangement is shown on Fig. 8A-B. Advantageously such allows the formation of a pocket for exudate, especially liquid stool, collection.

In an embodiment, shown in Fig.7A-C, the sheet-form (or sheet-like) layer **1** is further folded along the width **w** thereof in the upstream and/or downstream portions thereof (between which the intermediate portions lie). Advantages of this embodiment is to further provide added absorption and/or cushioning support to the back and/or belly region of the subject when wearing the article.

The cutting station may comprise one or more linear or shearing knives or one or more rotating drums comprising one or more knives on the perimetral surface thereof. Said knives may be timed for cutting the sheet either mechanically (via the use of cams and gears) or electromechanically via the use of servomotors and a virtual master reference acting as a metronome to provide the cutting signal. Nevertheless, other cutting means achieving the same end result of the process steps described herein may equally be used.

In an alternative and preferred embodiment, the cutting station may comprise one or more laser cutters arranged to cut the sheet-like layer, in the manner disclosed herein. An advantage of using laser as cutting means is that greater accuracy and range of shapes may be cut compared to mechanical knife means, as well as reduced change-over times between different consecutive products requiring different shapes to be cut.

The folding station may comprise a combination of stationary and kinetic lifting and/or folding means. For example, at or just prior to the folding position, the station may comprise protruding elements (preferably fixed and/or not moveable) arranged to lift the flaps as the sheet-like material is fed in the machine direction and downstream therefrom an actuated folding apparatus (e.g. servo-actuated, cam-driven or the like) arranged to force the lifted flaps to fold over the sheet-like layer. In an alternative embodiment, the fixed protrusions may be replaced with a blowing apparatus comprising nozzles arranged to eject compressed air (generally forming a reverse vacuum), preferably only, in the region of the flaps in order to lift them away from the plane of the sheet-like layer that is typically retained onto a conveyor belt by static force and/or vacuum suction. Although this is an exemplary embodiment of the folding station, it is apparent to a person skilled in the art that other such equipment known in the art may be used as long as the described folding is achieved.

Alternatively to the above, the cutting and folding may be carried at the same station and/or substantially concurrently.

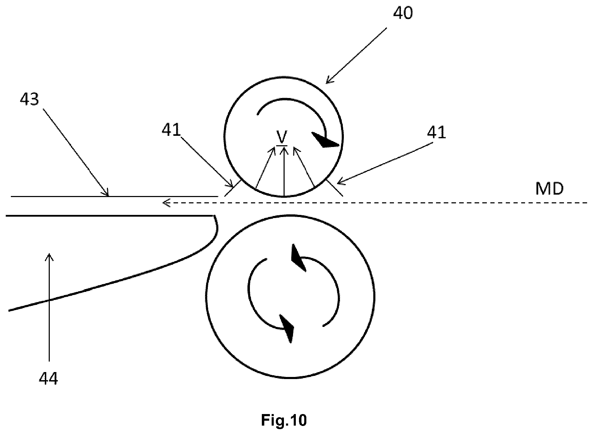

In an embodiment, shown in Fig.10, the cutting station may comprise a rotating roller **40** comprising one or more knives **41** shaped according to the needs described herein (e.g. straight, curved or angled about one or more planes). Preferably the roller **40** comprises at least two spaced apart knives **41** in a thickness direction (perpendicular to the machine direction **MD**) and said spacing being substantially equal to the width of the sheet-like layer, and preferably at least two spaced apart knives **41** in the machine direction **MD** being substantially equal to **d1**. The roller may comprise openings on an outer surface thereof in proximity to a receiving surface of the sheet-form layer, these openings may preferably be located between two consecutive knives **41**, and said openings being in fluid communication with an under pressure source such to form a vacuum force **V** arranged to lift the flap formed on the sheet-form layer substantially concurrently to the cutting thereof. Downstream said roller **40**, along the machine direction **MD**, a deflector **43** (stationary or kinetic) may be arranged to grip the lifted flap and fold it according to the embodiments described herein. A vacuum belt **44** may be arranged proximal to, and preferably below, the deflector **43** such that the folded sheet-like layer is conveyed further along the machine direction **MD**.

In an embodiment, a glue application step is carried out wherein an adhesive is applied on portions of the sheet-like layer (i.e. on the intermediate portion) where the flaps are to be joined thereto. Such step may be carried out via adhesive spray nozzles common in the art, such as those described in US5,474,540 and/or EP1593848A2. Such glue/adhesive applicator(s) may be positioned just prior (i.e. upstream) to the folding station or within the folding station provided that the adhesive is applied to the intermediate portion of the sheet-like layer prior to the actual folding of the flaps.

Alternatively, if mechanical bonding is used to join the flaps to the upper or lower surface of the sheet-like layer, such step is carried out downstream of the folding station typically within a sealing station (not shown) that may comprise an ultrasonic or heat sealing apparatus.

Whether glue and/or mechanical bonding is applied, according to the above embodiments, it is preferred that the bonding formed is temporary such that upon contact with liquid the flaps are released from their folded position, preferably to a semi-folded position. By "temporary" it is intended herein that the bonding strength is sufficiently strong to hold the flaps in a folded position during the process of making the absorbent cores and/or articles (i.e. in dry-state), and at least partly release when in contact with liquid exudates (i.e. in wetstate). The latter may be achieved by selecting the appropriate adhesive or use of mechanical bonding especially ultrasonic spot-bonding. An advantage of this arrangement is that a better basin formation is achieved especially in embodiments as will be described herein below wherein liquid stool collection is desired with reduced skin irritation.

Preferably, the sheet-form layer **1** comprises super absorbent polymer particles immobilized over or through the fibers that are arranged in the form of a sheet or web, preferably wherein said immobilization is attained by an adhesive or by mechanical locking. The mechanical locking may be achieved by applying ultrasound to force the particles to penetrate within the fibrous layer and lock/entangle with the fibers.

Preferably, the voids **13, 13'** form indentations in the sheet-form layer **1** extending in a widthwise direction thereof such that the intermediate portion **12** has a width that is less than that of the upstream and downstream portions **9, 10**, preferably to form one or more H-shaped, T-shaped or hourglass-shaped cores. Such further enables the desired anatomical shape.

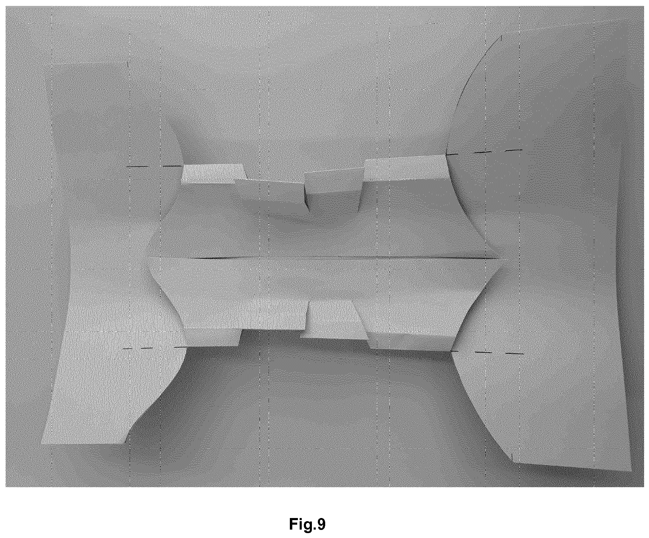

As shown in Fig. 9, a further plurality of cuts may be applied at each longitudinal edge thereof, after the intermediate flaps **8, 8'** are folded, such to form shaping sub-flaps arranged to conform with the anatomical shape of the body when worn. Such further cuts may be linear, curved or a combination thereof. The cuts may follow the transversal axis (i.e. straight cuts) along the width **w** of the sheet-like layer or core; or be at an angle α therefrom (i.e. angled cuts) typically being from 25° to 90°, more preferably from 30° to 85°, even more preferably from 35° to 80°, even more preferably from 40° to 75°, most preferably from 45° to 70°; or a combination thereof. Preferably a combination of both straight and angled cuts. An advantage is better shaping of the core is achieved and added flexibility aiding cup formation of the core and absorbent article.

In an embodiment (not shown), a process for making an absorbent article comprises the steps of: making an anatomical absorbent core according to the above described process steps (typically either in-line or preferably in a separate pre-production batch or process); providing a first web of material; providing a second web and/or film of material, wherein said first web is liquid permeable and said second web and/or film is liquid impermeable; conveying said core, said first web and said second web and/or film along a machine direction **MD** to a joining station; joining said core to said first web and said second web and/or film to form a laminate; optionally applying one or more add-ons to the laminate, said add-ons preferable selected from the group consisting of transversal and/or longitudinal cuffs, elastic or inelastic front and/or back side panels, female and/or male fasteners, and one or more elastic materials; optionally severing the laminate along an axis perpendicular to the machine direction **MD** to form a plurality of distinct absorbent articles.

In an embodiment, further layers (such as one or more acquisition distribution layers or ADL) are conveyed and subsequently joined between the first web of material and the core. Alternatively, and ADL may be conveyed and adhered in a facing relationship to the top or bottom surface of the sheet-like layer prior to the cutting and/or folding station such that upon cutting and folding said intermediate flaps said ADL is at least partly enwrapped by at least a portion of said flaps.

Conveying means and joining means as known in the art may be used to move the substrate and adhere them together to form the resulting laminates.

It is understood herein that any absorbent core and absorbent article arrangement resulting from embodiments discussed herein the context of the process of making may be directly generalized and applied, or can be combinable, to the core and article features and description according to the disclosure herein.

## Claims

1. A process for making an anatomical absorbent core (21) comprising the steps of:

    providing a continuous sheet-form layer (1) of absorbent material comprising a nonwoven carrier having natural and/or synthetic fibers and superabsorbent polymer particles immobilized therein and/or thereon, said sheet-form layer (1) comprising oppositely disposed top and bottom surfaces (2, 3), and first and second longitudinal sides (4, 5) and a width (w) extending between said sides (4, 5), and said sides (4, 5) extending substantially parallel to each other throughout the entire sheet-form layer (1) length extending along a machine direction (MD);
    advancing said sheet-form layer (1) along the machine direction (MD) to a cutting station (6);
    applying at least two cuts (7) to said sheet-form layer (1) at each of the first and second longitudinal sides (4, 5) along a cutting axis, wherein the each of said cuts (7) is spaced apart by a distance (d1) along a longitudinal axis running parallel to the machine direction (MD) and perpendicular to said width (w), said cuts (7) forming intermediate flaps (8, 8') between upstream and downstream portions (9, 10) of said sheet-form layer (1), wherein said cutting axis extends only parallel to said width (w) or at an and angle (α) therefrom such that no cuts are applied that extend parallel to the machine direction (MD); advancing said sheet-form layer (1) along a machine direction (MD) to a folding station (11); folding said intermediate flaps (8, 8') over the top or bottom surface (2, 3) of the sheet-form layer (1) to form an intermediate portion (12) comprising two or more voids (13, 13') oppositely disposed on each of the longitudinal sides (4, 5) and spaced apart by a distance (d2) along the width (w); optionally adhering the intermediate flaps (8, 8') to at least a portion of said top or bottom surface (2, 3); optionally severing the sheet-form layer (1) along the width (w) between each two consecutive voids (13, 13') positioned along the same first or second longitudinal side (4, 5) to form individual anatomical absorbent cores, and wherein the absorbent core (21) is free of fluff or distinct cellulosic fibers, and wherein each cut (7) applied on the first longitudinal side (4) has a first cut length (11) and each cut applied on the second longitudinal side (5) has a second cut length (l2) and wherein the following formula is satisfied:

$$l1 + l2 \geq d2$$

2. A process according to Claim 1 wherein the folded intermediate flaps (8, 8') substantially abut each other when in the folded position.

3. A process according to Claims 1 wherein the folded intermediate flaps (8, 8') substantially overlap one another when in the folded position.

4. A process according to any of the preceding Claims wherein the sheet-form layer (1) comprises super absorbent polymer particles immobilized over or through the fibers that are arranged in the form of a sheet or web, preferably wherein said immobilization is attained by an adhesive or by mechanical locking.

5. A process according to any of the preceding Claims wherein the voids (13, 13') form indentations in the sheet-form layer (1) extending in a widthwise direction thereof such that the intermediate portion (12) has a width that is less than that of the upstream and downstream portions (9, 10), preferably to form one or more H-shaped, T-shaped or hourglass-shaped cores.

6. A process for making an absorbent article comprising the steps of:

   making an anatomical absorbent core according to the process of Claims 1 to 5; providing a first web of material; providing a second web and/or film of material, wherein said first web is liquid permeable and said second web and/or film is liquid impermeable; conveying said core, said first web and said second web and/or film along a machine direction (MD) to a joining station; joining said core to said first web and said second web and/or film to form a laminate; optionally applying one or more add-ons to the laminate, said add-ons preferable selected from the group consisting of transversal and/or longitudinal cuffs, elastic or inelastic front and/or back side panels, female and/or male fasteners, and one or more elastic materials; optionally severing the laminate along an axis perpendicular to the machine direction (MD) to form a plurality of distinct absorbent articles.

7. An anatomical absorbent core (21), said core comprising:

   a front portion (29); a back portion (210); and an intermediate portion (12) extending between the front and back portions (29, 210), wherein said intermediate portion (12) is defined by at least, preferably only, two voids (13, 13') also positioned between the front and back portions (29, 210) and extending outboard of the intermediate portion (12) on opposite sides thereof, said voids (13,13') being oppositely disposed on each longitudinal sides (4, 5) of the intermediate portion (12) and spaced apart by a distance (d2) along the width (w) extending between said sides (4, 5), wherein the entire anatomical absorbent core (21) is capable of absorbing and retaining liquid therein and wherein front and back portions (29, 210) have a first and second absorption capacity per unit area respectively and the intermediate portion (12) has a third absorption capacity per unit area, as measured according to the test method described herein, wherein the third absorption capacity per unit area is at least about two times greater than the first absorption capacity per unit area, and preferably wherein said first and second absorption capacities are substantially the same, and wherein the absorbent core (21) comprises a sheet-form layer (1) comprising a nonwoven carrier having natural and/or synthetic fibers, and superabsorbent polymer particles immobilized therein and/or thereon and wherein the absorbent core (21) is free of fluff or distinct cellulosic fibers,

wherein the first longitudinal side (4) has a first cut length (l1) and the second longitudinal side (5) has a second cut length (l2) and wherein the following formula is satisfied:

$$l1 + l2 \geq d2$$

8.  An anatomical absorbent core (21) according to Claim 7 wherein the thickness of the intermediate portion (12) is about twice, or more, the thickness of the front and/or back portions (29, 210), as measured according to ISO 9073-2.

9.  An anatomical absorbent core (21) according to Claims 7 to 8 wherein said core is composed of a sheet-form layer (1) of absorbent material comprising natural and/or synthetic fibers, preferably selected from the group consisting of polyethylene fibers, polypropylene fibers, polyethylene terephthalate fibers, cellulose fibers, and mixtures thereof, and/or superabsorbent polymer, said sheet-form layer (1) comprising oppositely disposed top and bottom surfaces (2, 3), first and second longitudinal sides (4, 5) and a width (w) extending between said sides (4, 5), and wherein portions of the first and second longitudinal sides (4, 5) are folded over the top or bottom surface (2, 3) of the sheet-form layer (1) to form the intermediate portion (12), preferably wherein said portions of the first and second longitudinal sides (4, 5) are folded over said top or bottom surface (2, 3) a plurality of times, typically wherein said portions are the intermediate flaps (8, 8').

10. An anatomical absorbent core (21) according to Claims 7 to 9 having a substantially H-shaped, T-shaped or hourglass-shaped perimeter.

11. An anatomical absorbent core (21) according to Claims 9 to 10 wherein the sheet-form layer (1) comprises, preferably consists of, a nonwoven carrier comprising the fibers and superabsorbent polymer particles immobilized therein and/or thereon, preferably wherein the carrier comprises a nonwoven top layer, penetrable by the particles and a nonwoven bottom layer, said top layer being mechanically bonded by hydroentanglement to the bottom layer, wherein said carrier, the bottom layer and the top layer are hydrophilic, and wherein the bottom layer is porous with a pore size smaller than said particles.

12. An anatomical absorbent core (21) according to Claim 11 wherein the top layer is a carded nonwoven made of staple fibers which are hydro-entangled to the bottom layer and wherein the bottom layer is a spunbond meltblown spunbond fabric.

13. An anatomical absorbent core (21) according to any of the preceding Claims wherein said core is free of fluff or distinct cellulosic fibers.

14. An anatomical absorbent core (21) according to any of the preceding Claims wherein the intermediate flaps (8, 8') are folded such that an exudate-retaining basin is formed between the top or bottom surface (2, 3) of the intermediate portion (12) of the sheet-form layer (1) and the top or bottom surface of the intermediate flaps (8, 8') respectively.

15. An absorbent article (31) comprising an anatomical absorbent core (21) according to Claims 11 to 14 wherein the super absorbent polymer particles further have a size distribution and are dispersed in the carrier according to their particle size distribution gradient along a depth direction or z-direction of said absorbent core (21), and wherein the smaller super absorbent polymer particles are located on a garment-facing side of the absorbent article and the larger super absorbent polymer particles are located on the opposite side of the absorbent article on a body-facing side thereof.

**Patentansprüche**

1.  Verfahren zur Herstellung eines anatomischen absorbierenden Kerns (21), die folgenden Schritte umfassend:

    Bereitstellen einer durchgehenden folienförmigen Schicht (1) aus absorbierendem Material, einen Vliesträger umfassend, der Natur- und/oder Synthetikfasern und superabsorbierende Polymerpartikel, die darin und/oder daran immobilisiert sind, aufweist, wobei die folienförmige Schicht (1) eine Ober- und eine Unterseite (2, 3), die gegenüberliegend angeordnet sind, und eine erste und eine zweite Längsseite (4, 5) umfasst und eine Breite (w), die sich zwischen den Seiten (4, 5) erstreckt, und sich die Seiten (4, 5) über die gesamte Länge der folienförmigen Schicht (1), die sich entlang einer Maschinenrichtung (MD) erstreckt, im Wesentlichen parallel zueinander erstrecken,
    Befördern der folienförmigen Schicht (1) entlang der Maschinenrichtung (MD) zu einer Schneidestation (6),
    Setzen mindestens zweier Schnitte (7) an der folienförmigen Schicht (1) an jeder der ersten und der zweiten Längsseite (4, 5) entlang einer Schneidachse, wobei jeder der Schnitte (7) um eine Distanz (d1) entlang einer Längsachse, die parallel zu der Maschinenrichtung (MD) und senkrecht zu der Breite (w) verläuft, beabstandet ist, wobei die Schnitte (7) mittlere Klappen (8, 8') zwischen prozessaufwärtigen und prozessabwärtigen Abschnitten (9, 10) der folienförmigen Schicht (1) bilden, wobei sich die Schneidachse nur parallel zu der Breite (w) oder in einem Winkel ($\alpha$) dazu erstreckt, sodass keine Schnitte gesetzt werden, die sich parallel zu der Maschinenrichtung (MD) erstrecken,

Befördern der folienförmigen Schicht (1) entlang einer Maschinenrichtung (MD) zu einer Falzstation (11),

Falzen der mittleren Klappen (8, 8') über die Ober- oder Unterseite (2, 3) der folienförmigen Schicht (1), um einen mittleren Abschnitt (12) zu bilden, der zwei oder mehr Leerräume (13, 13') umfasst, die gegenüberliegend an jeder der Längsseiten (4, 5) angeordnet sind und um eine Distanz (d2) entlang der Breite (w) beabstandet sind,

optional Anheften der mittleren Klappen (8, 8') an mindestens einen Abschnitt der Ober- oder Unterseite (2, 3),

optional Trennen der folienförmigen Schicht (1) entlang der Breite (w) zwischen zwei aufeinanderfolgenden Leerräumen (13, 13'), die entlang derselben ersten oder zweiten Längsseite (4, 5) angeordnet sind, um einzelne anatomische absorbierende Kerne zu bilden,

und wobei der absorbierende Kern (21) frei von Flockenzellstoff oder einzelnen Zellulosefasern ist, und

wobei jeder Schnitt (7), der an der ersten Längsseite (4) gesetzt wird, eine erste Schnittlänge (l1) aufweist und jeder Schnitt, der an der zweiten Längsseite (5) gesetzt wird, eine zweite Schnittlänge (l2) aufweist und wobei die folgende Formel erfüllt ist:

$$l1 + l2 \geq d2$$

2. Verfahren nach Anspruch 1, wobei die gefalzten mittleren Klappen (8, 8') im Wesentlichen aneinander anliegen, wenn sie in der gefalzten Position sind.

3. Verfahren nach Anspruch 1, wobei die gefalzten mittleren Klappen (8, 8') einander im Wesentlichen überlappen, wenn sie in der gefalzten Position sind.

4. Verfahren nach einem der vorhergehenden Ansprüche, wobei die folienförmige Schicht (1) superabsorbierende Polymerpartikel umfasst, die über den Fasern, die in Form einer Folie oder einer Bahn angeordnet sind, oder durch diese hindurch immobilisiert sind, vorzugsweise wobei die Immobilisierung durch einen Klebstoff oder eine mechanische Blockierung erreicht wird.

5. Verfahren nach einem der vorhergehenden Ansprüche, wobei die Leerräume (13, 13') Einkerbungen in der folienförmigen Schicht (1) bilden, die sich in einer Breitenrichtung davon derart erstrecken, dass der mittlere Abschnitt (12) eine Breite aufweist, die kleiner ist als die der prozessaufwärtigen und prozessabwärtigen Abschnitte (9, 10), vorzugsweise um einen oder mehrere H-förmige, T-förmige oder sanduhrförmige Kerne zu bilden.

6. Verfahren zum Herstellen eines absorbierenden Artikels, die folgenden Schritte umfassend:

Herstellen eines anatomischen absorbierenden Kerns gemäß dem Verfahren nach den Ansprüchen 1 bis 5,
Bereitstellen einer ersten Bahn von Material,
Bereitstellen einer zweiten Bahn und/oder eines zweiten Films von Material, wobei die erste Bahn flüssigkeitsdurchlässig ist und die zweite Bahn und/oder der zweite Film flüssigkeitsundurchlässig ist,
Transportieren des Kerns, der ersten Bahn und der zweiten Bahn und/oder des zweiten Films entlang einer Maschinenrichtung (MD) zu einer Zusammenfügungsstation,
Zusammenfügen des Kerns mit der ersten Bahn und der zweiten Bahn und/oder dem zweiten Film, um ein Laminat zu bilden,
optional Anbringen eines oder mehrerer Zusätze an dem Laminat, wobei die Zusätze vorzugsweise aus der Gruppe ausgewählt werden, die aus Quer- und/oder Längsbündchen, elastischen oder unelastischen vorder- und/oder rückseitigen Feldern, aufnehmenden und/oder steckerartigen Befestigungsmitteln und einem oder mehreren elastischen Materialien besteht,
optional Trennen des Laminats entlang einer Achse, die senkrecht zu der Maschinenrichtung (MD) liegt, um mehrere einzelne absorbierende Artikel zu bilden.

7. Anatomischer absorbierender Kern (21), wobei der Kern Folgendes umfasst:

einen vorderen Abschnitt (29),
einen hinteren Abschnitt (210) und
einen mittleren Abschnitt (12), der sich zwischen dem vorderen und dem hinteren Abschnitt (29, 210) erstreckt, wobei der mittlere Abschnitt (12) definiert ist durch mindestens, vorzugsweise nur, zwei Leerräume (13, 13'), die ebenfalls zwischen dem vorderen und dem hinteren Abschnitt (29, 210) positioniert sind und sich von dem mittleren Abschnitt (12) an gegenüberliegenden Seiten davon auswärts erstrecken, wobei die Leerräume (13, 13') gegenüberliegend an jeder Längsseite (4, 5) des mittleren Abschnitts (12) angeordnet und um eine Distanz (d2) entlang der Breite (w), die sich zwischen den Seiten (4, 5) erstreckt, beabstandet sind, wobei der gesamte anatomische absorbierende Kern (21) in der Lage ist, Flüssigkeit darin zu absorbieren und zu halten, und wobei der vordere und der hintere Abschnitt (29, 210) ein erstes beziehungsweise ein zweites Absorptionsvermögen pro Flächeneinheit aufweisen und der mittlere Abschnitt (12) ein drittes Absorptionsvermögen pro Flächeneinheit aufweist, gemessen gemäß dem hierin beschriebenen Testverfahren, wobei das dritte Absorptionsvermögen pro Flächeneinheit mindestens ungefähr zweimal größer ist als das erste Absorptionsvermögen pro Flächeneinheit und vorzugsweise wobei das erste und das zweite Absorptionsvermögen im Wesentlichen gleich sind, und wobei der absorbierende Kern (21) eine folienförmige Schicht (1) umfasst, die einen Vliesträger umfasst, der Natur- und/oder Synthetikfasern und superabsorbierende Polymerpartikel aufweist, die darin und/oder daran immobilisiert sind, und wobei der absorbierende Kern (21) frei von Flockenzellstoff oder einzelnen Zellulosefasern ist, wobei die erste Längsseite (4) eine erste Schnittlänge (11) aufweist und die zweite Längsseite (5) eine zweite Schnittlänge (l2) aufweist und wobei die folgende Formel erfüllt ist:

$$l1 + l2 \geq d2$$

8. Anatomischer absorbierender Kern (21) nach Anspruch 7, wobei die Dicke des mittleren Abschnitts (12) ungefähr das Zweifache oder mehr der Dicke des vorderen und/oder des hinteren Abschnitts (29, 210) beträgt, gemessen nach ISO 9073-2.

9. Anatomischer absorbierender Kern (21) nach den Ansprüchen 7 bis 8, wobei der Kern aus einer folienförmigen Schicht (1) aus absorbierendem Material besteht, das Natur- und/oder Synthetikfasern, die vorzugsweise aus der Gruppe ausgewählt sind, die aus Polyethylenfasern, Polypropylenfasern, Polyethylenterephthalatfasern, Cellulosefasern und Mischungen daraus besteht, und/oder superabsorbierende Polymerpartikel umfasst, wobei die folienförmige Schicht (1) eine Ober- und eine Unterseite (2, 3), die gegenüberliegend angeordnet sind, eine erste und eine zweite Längsseite (4, 5) und eine Breite (w) umfasst, die sich zwischen den Seiten (4, 5) erstreckt, und wobei Abschnitte der ersten und der zweiten Längsseite (4, 5) über die Ober- oder Unterseite (2, 3) der folienförmigen Schicht (1) gefalzt sind, um einen mittleren Abschnitt (12) zu bilden, vorzugsweise wobei die Abschnitte der ersten und der zweiten Längsseite (4, 5) mehrmals über die Ober- oder Unterseite (2, 3) gefalzt sind, typischerweise wobei die Abschnitte die mittleren Klappen (8, 8') sind.

10. Anatomischer absorbierender Kern (21) nach den Ansprüchen 7 bis 9, einen im Wesentlichen H-förmigen, T-förmigen oder sanduhrförmigen Umfang aufweisend.

11. Anatomischer absorbierender Kern (21) nach den Ansprüchen 9 bis 10, wobei die folienförmige Schicht (1) einen Vliesträger umfasst, vorzugsweise daraus besteht, der die Fasern und superabsorbierende Polymerpartikel umfasst, die darin und/oder daran immobilisiert sind, vorzugsweise wobei der Träger eine obere Vliesschicht, die von den Partikeln durchdrungen werden kann, und eine untere Vliesschicht umfasst, wobei die obere Schicht durch Wasserstrahlverfestigung mechanisch an die untere Schicht gebunden ist, wobei der Träger, die untere Schicht und die obere Schicht hydrophil sind und wobei die untere Schicht porös mit einer Porengröße ist, die kleiner ist als die Partikel.

12. Anatomischer absorbierender Kern (21) nach Anspruch 11, wobei die obere Schicht ein kardiertes Vlies ist, das aus Stapelfasern besteht, die an die untere Schicht wasserstrahlverfestigt ist, und wobei die untere Schicht ein Spunbond-Meltblown-Spunbond-Flächengebilde ist.

13. Anatomischer absorbierender Kern (21) nach einem der vorhergehenden Ansprüche, wobei der Kern frei von Flockenzellstoff oder einzelnen Zellulosefasern ist.

14. Anatomischer absorbierender Kern (21) nach einem der vorhergehenden Ansprüche, wobei die mittleren Klappen (8, 8') derart gefalzt sind, dass ein Absonderungen haltendes Becken zwischen der Ober- oder Unterseite (2, 3) des mittleren Abschnitts (12) der folienförmigen Schicht (1) und der Oberbeziehungsweise der Unterseite der mittleren Klappen (8, 8') gebildet ist.

15. Absorbierender Artikel (31), umfassend einen anatomischen absorbierenden Kern (21) nach den Ansprüchen 11 bis 14, wobei die superabsorbierenden Polymerpartikel ferner eine Größenverteilung aufweisen und gemäß ihrem Gradienten der Partikelgrößenverteilung entlang einer Tiefenrichtung oder Z-Richtung des absorbierende Kerns (21) in dem Träger verteilt sind, und wobei sich die kleineren superabsorbierenden Polymerpartikel auf einer zur Kleidung weisenden Seite des absorbierenden Artikels befinden und sich die größeren superabsorbierenden Polymerpartikel auf der gegenüberliegenden Seite des absorbierenden Artikels auf einer zum Körper weisenden Seite desselben befinden.

**Revendications**

**1.** Procédé de fabrication d'un noyau absorbant anatomique (21) comprenant les étapes de :

fourniture d'une couche en forme de feuille continue (1) de matériau absorbant comprenant un support non tissé ayant des fibres naturelles et/ou synthétiques et des particules de polymère superabsorbant immobilisées dans et/ou sur celles-ci, ladite couche en forme de feuille (1) comprenant des surfaces supérieure et inférieure (2, 3) disposées à l'opposé, et des premier et second côtés longitudinaux (4, 5) et une largeur (w) s'étendant entre lesdits côtés (4, 5), et lesdits côtés (4, 5) s'étendant sensiblement parallèlement l'un à l'autre sur la totalité de la longueur de la couche en forme de feuille (1) s'étendant le long d'un sens machine (SM) ;
progression de ladite couche en forme de feuille (1) le long du sens machine (SM) vers un poste de coupe (6) ;
application d'au moins deux découpes (7) à ladite couche en forme de feuille (1) au niveau de chacun des premier et second côtés longitudinaux (4, 5) le long d'un axe de coupe, dans lequel chacune desdites découpes (7) est espacée d'une distance (d1) le long d'un axe longitudinal s'étendant parallèlement au sens machine (SM) et perpendiculairement à ladite largeur (w), lesdites découpes (7) formant des rabats intermédiaires (8, 8') entre des parties amont et aval (9, 10) de ladite couche en forme de feuille (1), dans lequel ledit axe de coupe s'étend uniquement parallèlement à ladite largeur (w) ou à un angle ($\alpha$) par rapport à celle-ci de telle sorte à n'appliquer aucune découpe qui s'étend parallèlement au sens machine (SM) ;
progression de ladite couche en forme de feuille (1) le long d'un sens machine (SM) vers un poste de pliage (11) ;
pliage desdits rabats intermédiaires (8, 8') sur la surface supérieure ou inférieure (2, 3) de la couche en forme de feuille (1) pour former une partie intermédiaire (12) comprenant deux vides (13, 13') ou plus disposés à l'opposé sur chacun des côtés longitudinaux (4, 5) et espacés d'une distance (d2) le long de la largeur (w) ;
adhésion éventuelle des rabats intermédiaires (8, 8') à au moins une partie de ladite surface supérieure ou inférieure (2, 3) ;
sectionnement éventuel de la couche en forme de feuille (1) le long de la largeur (w) entre chaque deux vides (13, 13') consécutifs positionnés le long du même premier ou second côté longitudinal (4, 5) pour former des noyaux absorbants anatomiques individuels,
et dans lequel le noyau absorbant (21) est exempt de peluches ou de fibres cellulosiques distinctes, et
dans lequel chaque découpe (7) appliquée sur le premier côté longitudinal (4) a une première longueur de découpe (l1) et chaque découpe appliquée sur le second côté longitudinal (5) a une seconde longueur de découpe (l2) et dans lequel la formule suivante est satisfaite :

$$l1 + l2 \geq d2$$

**2.** Procédé selon la revendication 1, dans lequel les rabats intermédiaires (8, 8') pliés viennent sensiblement en butée l'un contre l'autre lorsqu'ils sont dans la position pliée.

**3.** Procédé selon la revendication 1, dans lequel les rabats intermédiaires (8, 8') pliés se chevauchent sensiblement l'un l'autre lorsqu'ils sont dans la position pliée.

**4.** Procédé selon l'une quelconque des revendications précédentes, dans lequel la couche en forme de feuille (1) comprend des particules de polymère superabsorbant immobilisées sur ou à travers les fibres qui sont agencées sous la forme d'une feuille ou d'une bande, de préférence dans lequel ladite immobilisation est obtenue par un adhésif ou par une liaison mécanique.

**5.** Procédé selon l'une quelconque des revendications précédentes, dans lequel les vides (13, 13') forment des indentations dans la couche en forme de feuille (1) s'étendant dans un sens de la largeur de celle-ci de telle sorte que la partie intermédiaire (12) a une largeur qui est inférieure à celle des parties amont et aval (9, 10), de préférence pour former un ou plusieurs noyaux en forme de H, en forme de T ou en forme de sablier.

**6.** Procédé de fabrication d'un article absorbant comprenant les étapes de :

fabrication d'un noyau absorbant anatomique selon le procédé des revendications 1 à 5 ;
fourniture d'une première bande e matériau ;
fourniture d'une seconde bande et/ou d'un second film de matériau, dans lequel ladite première bande est perméable aux liquides et ladite seconde bande et/ou ledit second film est imperméable aux liquides ;
acheminement dudit noyau, de ladite première bande et de ladite seconde bande et/ou dudit second film le long d'un sens machine (SM) vers un poste d'assemblage ;

assemblage dudit noyau à ladite première bande et à ladite seconde bande et/ou audit second film pour former un stratifié ;

application éventuelle d'un ou plusieurs ajouts au stratifié, lesdits ajouts étant de préférence sélectionnés dans le groupe consistant en des manchons transversaux et/ou longitudinaux, des panneaux latéraux avant et/ou arrière élastiques ou inélastiques, des fixations femelles et/ou males et un ou plusieurs matériaux élastiques ;

sectionnement éventuel du stratifié le long d'un axe perpendiculaire au sens machine (SM) pour former une pluralité d'articles absorbants distincts.

7. Noyau absorbant anatomique (21), ledit noyau comprenant :

une partie avant (29) ;
une partie arrière (210) ; et
une partie intermédiaire (12) s'étendant entre les parties avant et arrière (29, 210), dans lequel ladite partie intermédiaire (12) est définie par au moins, de préférence uniquement, deux vides (13, 13') également positionnés entre les parties avant et arrière (29, 210) et s'étendant à l'extérieur de la partie intermédiaire (12) sur des côtés opposés de celle-ci, lesdits vides (13, 13') étant disposés à l'opposé sur chaque côté longitudinal (4, 5) de la partie intermédiaire (12) et espacés d'une distance (d2) le long de la largeur (w) s'étendant entre lesdits côtés (4, 5),

dans lequel la totalité du noyau absorbant anatomique (21) est capable d'absorber et de retenir du liquide à l'intérieur de celui-ci et dans lequel les parties avant et arrière (29, 210) ont une première et une deuxième capacité d'absorption par surface unitaire respectivement et la partie intermédiaire (12) a une troisième capacité d'absorption par surface unitaire, telles que mesurées selon la méthode d'essai décrite dans les présentes, dans lequel la troisième capacité d'absorption par surface unitaire est au moins environ deux fois plus grande que la première capacité d'absorption par surface unitaire, et de préférence dans lequel lesdites première et deuxième capacités d'absorption sont sensiblement identiques, et dans lequel le noyau absorbant (21) comprend une couche en forme de feuille (1) comprenant un support non tissé ayant des fibres naturelles et/ou synthétiques et des particules de polymère superabsorbant immobilisées dans et/ou sur celles-ci et dans lequel le noyau absorbant (21) est exempt de peluches ou de fibres cellulosiques distinctes, dans lequel le premier côté longitudinal (4) a une première longueur de découpe (l1) et le second côté longitudinal (5) a une seconde longueur de découpe (l2) et dans lequel la formule suivante est satisfaite :

$$l1 + l2 \geq d2$$

8. Noyau absorbant anatomique (21) selon la revendication 7, dans lequel l'épaisseur de la partie intermédiaire (12) est d'environ deux fois, ou plus, l'épaisseur des parties avant et/ou arrière (29, 210), telle que mesurée selon ISO 9073-2.

9. Noyau absorbant anatomique (21) selon les revendications 7 à 8, dans lequel ledit noyau est composé d'une couche en forme de feuille (1) de matériau absorbant comprenant des fibres naturelles et/ou synthétiques, de préférence sélectionnées dans le groupe consistant en des fibres de polyéthylène, des fibres de polypropylène, des fibres de polyéthylène téréphtalate, des fibres de cellulose et des mélanges de celles-ci, et/ou un polymère superabsorbant, ladite couche en forme de feuille (1) comprenant des surface supérieure et inférieure (2, 3) disposées à l'opposé, des premier et second côtés longitudinaux (4, 5) et une largeur (w) s'étendant entre lesdits côtés (4, 5), et dans lequel des parties des premier et second côtés longitudinaux (4, 5) sont pliées sur la surface supérieure ou inférieure (2, 3) de la couche en forme de feuille (1) pour former la partie intermédiaire (12), de préférence dans lequel lesdites parties des premier et second côtés longitudinaux (4, 5) sont pliées sur ladite surface supérieure ou inférieure (2, 3) une pluralité de fois, d'ordinaire dans lequel lesdites parties sont les rabats intermédiaires (8, 8').

10. Noyau absorbant anatomique (21) selon les revendications 7 à 9 ayant un périmètre de forme sensiblement en H, en T ou en sablier.

11. Noyau absorbant anatomique (21) selon les revendications 9 à 10, dans lequel la couche en forme de feuille (1) comprend, de préférence consiste en, un support non tissé comprenant les fibres et particules de polymère superabsorbant immobilisées dans et/ou sur celles-ci, de préférence dans lequel le support comprend une couche supérieure non tissée, pénétrable par les particules et une couche inférieure non tissée, ladite couche supérieure étant liée mécaniquement par hydroliage à la couche inférieure, dans lequel ledit support, la couche inférieure et la couche supérieure sont hydrophiles, et dans lequel la couche inférieure est poreuse avec une taille de pore plus petite que lesdites particules.

12. Noyau absorbant anatomique (21) selon la revendication 11, dans lequel la couche supérieure est un non-tissé cardé composé de fibres discontinues qui sont hydroliées à la couche inférieure et dans lequel la couche inférieure est un tissu SMS (filé lié + soufflé à l'état fondu +filé lié).

**13.** Noyau absorbant anatomique (21) selon l'une quelconque des revendications précédentes, dans lequel ledit noyau est exempt de peluches ou de fibres cellulosiques distinctes.

**14.** Noyau absorbant anatomique (21) selon l'une quelconque des revendications précédentes, dans lequel les rabats intermédiaires (8, 8') sont pliés de telle sorte qu'une cuvette retenant des exsudats est formée entre la surface supérieure ou inférieure (2, 3) de la partie intermédiaire (12) de la couche en forme de feuille (1) et la surface supérieure ou inférieure des rabats intermédiaires (8, 8') respectivement.

**15.** Article absorbant (31) comprenant un noyau absorbant anatomique (21) selon les revendications 11 à 14, dans lequel les particules de polymère superabsorbant ont en outre une distribution granulométrique et sont dispersées dans le support selon leur gradient de distribution granulométrique le long d'un sens de profondeur ou d'un sens z dudit noyau absorbant (21), et dans lequel les particules de polymère superabsorbant plus petites sont situées sur un côté orienté vers le vêtement de l'article absorbant et les particules de polymère superabsorbant plus grandes sont situées sur le côté opposé de l'article absorbant sur un côté de celui-ci orienté vers le corps.

FIG. 1

FIG. 2

FIG. 3A

FIG. 3B

FIG. 3C

8'                          8

**FIG. 4**

31

FIG. 5

Fig.6A

Fig.6B

Fig.6C

Fig.6D

Fig.6E

Fig.6F

Fig.7A

Fig.7B

Fig.7C

Fig.8A

Fig.8B

Fig.9

Fig.10

**REFERENCES CITED IN THE DESCRIPTION**

**Patent documents cited in the description**

- US 5151092 A
- WO 2008155699 A
- US 20080312621 A
- US 20080312622 A
- US 2013331806 A
- US 2014163501 A
- GB 2174037 A
- US 2852026 A
- EP 3190216 A1
- US 5474540 A
- EP 1593848 A2